(19) Europäisches Patentamt
European Patent Office
Office européen des brevets

(11) EP 4 238 583 A1

(12) **EUROPEAN PATENT APPLICATION**
published in accordance with Art. 153(4) EPC

(43) Date of publication:
**06.09.2023 Bulletin 2023/36**

(21) Application number: **21886729.9**

(22) Date of filing: **25.10.2021**

(51) International Patent Classification (IPC):
***A61K 49/00*** (2006.01) ***A61K 8/41*** (2006.01)
***A61K 8/9789*** (2017.01) ***A61K 8/44*** (2006.01)
***A61K 8/49*** (2006.01) ***A61K 8/9728*** (2017.01)
***A61K 8/98*** (2006.01)

(52) Cooperative Patent Classification (CPC):
**A61K 8/34; A61K 8/41; A61K 8/44; A61K 8/49; A61K 8/60; A61K 8/66; A61K 8/67; A61K 8/9728; A61K 8/9789; A61K 8/98; A61K 49/00**

(86) International application number:
**PCT/KR2021/015034**

(87) International publication number:
**WO 2022/092735 (05.05.2022 Gazette 2022/18)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **29.10.2020 KR 20200142413**
**29.10.2020 KR 20210067761**
**19.07.2021 KR 20210094207**

(71) Applicants:
- **Kim, Jin Wang**
  **Gangnam-gu, Seoul 06017 (KR)**
- **Lee, Jung Ok**
  **Gangnam-gu, Seoul 06017 (KR)**
- **Kim, Hyun Jin**
  **Gangnam-gu, Seoul 06017 (KR)**
- **Kim, Hyun Woo**
  **Gangnam-gu, Seoul 06017 (KR)**

(72) Inventors:
- **Kim, Jin Wang**
  **Gangnam-gu, Seoul 06017 (KR)**
- **Lee, Jung Ok**
  **Gangnam-gu, Seoul 06017 (KR)**
- **Kim, Hyun Jin**
  **Gangnam-gu, Seoul 06017 (KR)**
- **Kim, Hyun Woo**
  **Gangnam-gu, Seoul 06017 (KR)**

(74) Representative: **Global IP Europe Patentanwaltskanzlei**
**Pfarrstraße 14**
**80538 München (DE)**

# (54) COMPOSITION CONTAINING 5-AMINOLEVULINIC ACID HYDROCHLORIDE

(57) The present invention provides a composition containing 5-aminolevulinic acid hydrochloride, the composition comprising 5-aminolevulinic acid hydrochloride, fermented Codonopsis lanceolata extract, a Cordyceps militaris extract, krill oil, polydeoxyribonucleotide sodium, propylene glycol, hyaluronidase, L-carnitine, caffeine, Origanum vulgare leaf oil, ascorbic acid (vitamin C), allantoin, cetyl alcohol and mineral water.

FIG. 1

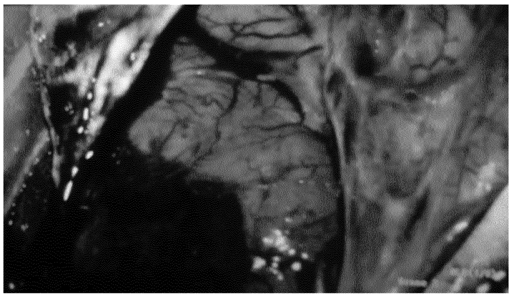

EP 4 238 583 A1

## Description

[Field of Invention]

The present invention relates to a composition including 5-aminolevulinic acid hydrochloride, and more particularly to a composition including 5-aminolevulinic acid hydrochloride that is a material used in photodynamic therapy (PDT), thereby being capable of being used as an oral drug composition for fluorescence-induced malignant tissue visualization or a cosmetic composition for acne skin improvement.

In addition, the present invention relates to a composition including 5-aminolevulinic acid hydrochloride, and more particularly to a composition including 5-aminolevulinic acid hydrochloride that is a material used for photodynamic therapy and diagnosis (PDT & PDD), thereby being capable of being used as an oral drug composition for fluorescence-induced malignant tissue visualization and, accordingly, being capable of being used for early diagnosis of tumors by accumulating in cancer cells or cancer tissues to show fluorescence or phosphorescence and maximizing the efficiency of diagnosis and treatment of cancer.

[Background of Invention]

Photodynamic therapy (PDT) is a technology that uses a photo-sensitizer (hereinafter referred to as "photo-sensitizer") to treat incurable diseases, such as cancer, or acne without surgery. Such photodynamic therapy (PDT) has been actively researched since the early 20th century, and is currently used in the diagnosis and treatment of cancer, allogeneic and autologous bone marrow transplantation, resolution of host-sponsored transplantation reactions by photopheresis, antibiotics, treatment of infectious diseases such as AIDS, organ and skin transplantation, treatment of arthritis, treatment of spinal muscular atrophy, and the like. In addition, the photodynamic therapy (PDT) is used to strengthen immunity or to control and regulate immune responses, and the application range thereof is gradually expanding, such as in regenerative medicine, biosensors, and optogenetics.

In particular, PDT, which is used for cancer treatment, generates singlet oxygen or free radicals through a chemical reaction between abundant oxygen in the body and external light when a photosensitizer that is sensitive to light is injected into the body and irradiated with light from the outside. PDT is a treatment using the principle that the singlet oxygen or the free radical induces and destroys apoptosis of various lesions or cancer cells.

As photosensitizers currently used in PDT therapy, a porphyrin derivative, chlorin, bacteriochlorin, phthalocyanine, a 5-aminolevulinic acid derivative, porfimer sodium, and the like are known.

The cyclic tetrapyrrole derivative, as a photosensitizer, is not only selectively accumulated in cancer cells, but also shows fluorescence or phosphorescence due to the characteristics of the compound, so it can be utilized as an early diagnosis reagent for tumors. In addition, since metalloporphyrin, in which metal is bound to the inside of cyclic tetrapyrrole, exhibits various properties depending on the type of metal bound, metalloporphyrin is used as a contrasting agent in magnetic resonance imaging (MRI) for early diagnosis of tumor cells such as cancer cells. The most widely known photosensitizer, 5-aminolevulinic acid derivative, has the advantages of simple use, low molecular weight, relatively easy penetration into the skin, and safety with few side effects.

In addition, photodynamic therapy has the advantages of being able to selectively remove only cancer cells while preserving normal cells, eliminating the risk of general anesthesia in most cases, and being able to operate with only simple local anesthesia.

(Related Art Documents)

(Patent Document 1) Korean Patent No. 10-1322259 (Registered on October 21, 2013)
(Patent Document 2) Korean Patent No. 10-1638664 (Registered on July 05, 2016)
(Patent Document 3) Korean Patent No. 10-1135147 (Registered on April 03, 2012)
(Patent Document 4) Korean Patent No. 10-1622031 (Registered on May 11, 2016)
(Patent Document 5) Korean Patent No. 10-1659855 (Registered on September 20, 2016)
(Patent Document 6) Korean Patent No. 10-1183732 (Registered on September 11, 2012)

[Summary of Invention]

[Technical Problem to be Solved]

Therefore, the present invention has been made in view of the above problems, and it is one object of the

present invention to provide a composition including 5-aminolevulinic acid hydrochloride that is a material used in photodynamic therapy (PDT), the composition being capable of being used as an oral drug composition for fluorescence-induced malignant tissue visualization or as a cosmetic composition for acne skin improvement.

It is another object of the present invention to provide a cosmetic composition including 5-aminolevulinic acid hydrochloride that is a material used in photodynamic therapy (PDT), the cosmetic composition being capable of suppressing inflammatory reactions occurring in skin tissue while minimizing skin irritation and of effectively improving acne skin and skin troubles.

It is yet another object of the present invention to provide an oral drug composition for fluorescence-induced malignant tissue visualization including 5-aminolevulinic acid hydrochloride that is a material used in photodynamic therapy and diagnosis (PDT & PDD), the oral drug composition being capable of being used for early diagnosis of tumors by accumulating in cancer cells or cancer tissues to show fluorescence or phosphorescence and of maximizing the efficiency of diagnosis and treatment of cancer.

It will be understood that technical problems to be solved of the present invention are not limited to those referred above and other non-referred technical problems will be clearly understood by those skilled in the art from disclosures below.

[Technical Solution]

A composition including 5-aminolevulinic acid hydrochloride according to the present invention is used as an oral drug composition for fluorescence-induced malignant tissue visualization.

The 5-aminolevulinic acid hydrochloride is orally administered, and then absorbed to make a tumor site emit a fluorescent color so that the normal cell tissue and a tumor cell site are distinguished. 5-aminolevulinic acid hydrochloride included in the oral drug composition for fluorescence-induced malignant tissue visualization may be administered in an amount of 18 to 20 mg per patient's body weight and, for surgery, may be orally administered 2 to 4 hours before induction of anesthesia.

30 mg of 5-aminolevulinic acid hydrochloride may be contained per 1 mL of a solution by dissolving 1.5 g of 5-aminolevulinic acid hydrochloride in 50 mL of purified water.

In addition, the composition including 5-aminolevulinic acid hydrochloride according to the present invention may include 5-aminolevulinic acid hydrochloride, fermented black codonopsis lanceolata extract, cordyceps extract, krill oil, polydeoxyribonucleotide sodium, propylene glycol, hyaluronidase, L-carnitine, caffeine, origanum vulgare leaf oil, ascorbic acid (vitamin C), allantoin, cetyl alcohol and mineral water, thereby being used as a cosmetic composition for acne skin improvement.

The composition may include 8 to 12 % by weight of 5-aminolevulinic acid hydrochloride, 0.1 to 2.5 % by weight of fermented black codonopsis lanceolata extract, 1 to 3 % by weight of cordyceps extract, 0.5 to 1.5 % by weight of krill oil, 1 to 2 % by weight of polydeoxyribonucleotide sodium, 10 to 20 % by weight of propylene glycol, 1 to 3 % by weight of hyaluronidase, 3 to 5 % by weight of L-carnitine, 2 to 4 % by weight of caffeine, 0.1 to 1.0 % by weight of origanum vulgare leaf oil, 1 to 5 % by weight of ascorbic acid (vitamin C), 1 to 3 % by weight of allantoin, 5 to 10 % by weight of cetyl alcohol and a balance of mineral water.

In addition, the composition including 5-aminolevulinic acid hydrochloride may include 5-aminolevulinic acid hydrochloride, indole-3-acetic acid (IAA), polyethyleneimine, self-assembled ligand particles, purified water, a carrier, glycerin fatty acid ester and an emulsifier.

The composition may include 30 to 50 parts by weight of 5-aminolevulinic acid hydrochloride, 10 to 20 parts by weight of indole-3-acetic acid (IAA), 1 to 5 parts by weight of polyethyleneimine, 5 to 15 parts by weight of self-assembled ligand particles, 10 to 20 parts by weight of purified water, 10 to 20 parts by weight of a carrier, 20 to 30 parts by weight of glycerin fatty acid ester and 10 to 20 parts by weight of an emulsifier based on a weight ratio.

The composition for photodynamic therapy including 5-aminolevulinic acid hydrochloride, indole-3-acetic acid (IAA), polyethyleneimine, self-assembled ligand particles, a carrier, glycerin fatty acid ester and an emulsifier may be administered by drinking in an amount of 35 mg/kg per patient's body weight (kg), and after 6 hours, light in a wavelength range of 660 to 680 nm may be irradiated to a tumor site of a patient to be photoactivated in vivo.

Details of other embodiments are included in the detailed description.

[Effect of Invention]

A composition including 5-aminolevulinic acid hydrochloride according to the present invention can be used as an oral drug composition for fluorescence-induced malignant tissue visualization or as a cosmetic composition for acne skin improvement by including 5-aminolevulinic acid hydrochloride that is a material used in photodynamic therapy (PDT) .

In addition, the composition including 5-aminolevulinic acid hydrochloride according to the present invention can be used as a cosmetic composition by including 5-aminolevulinic acid hydrochloride that is a material used in

photodynamic therapy (PDT), thereby being capable of suppressing inflammatory reactions occurring in skin tissue while minimizing skin irritation and of effectively improving acne skin and skin troubles.

In addition, the composition including 5-aminolevulinic acid hydrochloride is used as an oral drug composition for fluorescence-induced malignant tissue visualization by including 5-aminolevulinic acid hydrochloride that is a material used in photodynamic therapy and diagnosis (PDT & PDD), thereby being capable of being used for early diagnosis of tumors by accumulating in cancer cells or cancer tissues to show fluorescence or phosphorescence and of maximizing the efficiency of diagnosis and treatment of cancer.

It will be fully understood that embodiments of the technical idea of the present invention can provide various effects not specifically mentioned.

[Brief Description of Drawings]

FIG. 1 is a photograph illustrating a patient's brain tumor area emitting a fluorescent color when a doctor performed surgery 6 hours after a patient drank a composition including 5-aminolevulinic acid hydrochloride according to the present invention.

FIG. 2 is a graph illustrating a change in the amount of sebum excretion after 5 days of applying cosmetic compositions for acne skin improvement according to an example and a comparative example to the subject's acne skin.

FIGS. 3A, 4A, 5A, and 6A are photographs illustrating the subject's acne skin condition before applying the cosmetic composition prepared according to the example.

FIGS. 3B, 4B, 5B and 6B are photographs illustrating the subject's acne skin condition after applying the cosmetic composition prepared according to the example once a day for 20 days.

FIG. 7A is a photograph illustrating the location of a patient's brain tumor before the patient drinks a composition for photodynamic therapy including 5-aminolevulinic acid hydrochloride according to the present invention.

FIG. 7B is a photograph illustrating a state in which a patient's brain tumor area emits a fluorescent color when a doctor performs an operation 6 hours after the patient drank the composition for photodynamic therapy including 5-aminolevulinic acid hydrochloride according to the present invention.

[Best Mode for Carrying Out the Invention]

The advantages and features of the present invention, and how to achieve them, will become clear with reference to the detailed description of the embodiments below. However, the present invention is not limited to the embodiments described herein and may be embodied in other forms. Rather, the embodiments introduced herein are provided so that the disclosed content is to be thorough and complete and the idea of the present invention is sufficiently conveyed to those skilled in the art.

The terminology used herein is for the purpose of describing particular embodiments only and is not intended to be limiting of the inventive concept. The expression of singularity in the present specification includes the expression of plurality unless clearly specified otherwise in the context.

Unless otherwise defined, all terms (including technical and scientific terms) used herein have the same meaning as commonly understood by one of ordinary skill in the art to which this inventive concept belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

Hereinafter, a preferred embodiment of a composition including 5-aminolevulinic acid hydrochloride according to the present invention is described in detail with reference to the attached drawings.

A composition including 5-aminolevulinic acid hydrochloride according to an embodiment of the technical idea of the present invention may be an oral drug composition for fluorescence-induced malignant tissue visualization.

That is, the composition including 5-aminolevulinic acid hydrochloride according to an embodiment of the technical idea of the present invention includes 5-aminolevulinic acid hydrochloride that is a material used in photodynamic therapy (PDT), thereby being capable of being used as an oral drug composition for fluorescence-induced malignant tissue visualization.

The 5-aminolevulinic acid hydrochloride makes a tumor area emit fluorescent color after a patient drinks it, so that the tumor area can be removed while protecting normal tissue as much as possible during surgery.

The 5-aminolevulinic acid hydrochloride is a material that can be used for photodynamic therapy (PDT). Here, PDT is a technique for treating incurable diseases such as cancer without surgery using a photo-sensitizer (hereinafter referred to as a "photosensitizer").

The photodynamic therapy (PDT) has been actively researched since the early 20th century, and is currently

used in the diagnosis and treatment of cancer, allogeneic and autologous bone marrow transplantation, resolution of host-sponsored transplantation reactions by photopheresis, antibiotics, treatment of infectious diseases such as AIDS, organ and skin transplantation, treatment of arthritis, treatment of spinal muscular atrophy, and the like.

In addition, the photodynamic therapy (PDT) is used to strengthen immunity or to control and regulate immune responses, and the application range thereof is gradually expanding to regenerative medicine, biosensors, optogenetics, and the like.

In particular, PDT, which is used for cancer treatment, generates singlet oxygen or free radicals through a chemical reaction between abundant oxygen in the body and external light when a photosensitizer that is sensitive to light is injected into the body and irradiated with light from the outside. PDT is a treatment using the principle that the singlet oxygen or the free radical induces and destroys apoptosis of various lesions or cancer cells.

As photosensitizers currently used in PDT therapy, a porphyrin derivative, chlorin, bacteriochlorin, phthalocyanine, a 5-aminolevulinic acid derivative, porfimer sodium, and the like are known.

The 5-aminolevulinic acid hydrochloride is a C-5 aliphatic compound represented by [Formula 1] below and is one of intermediates found in the process of tetrapyrrole biosynthesis (prophyrin, chlorophyll, heme, vitamin B12, etc.) of various organisms:

[Formula 1]

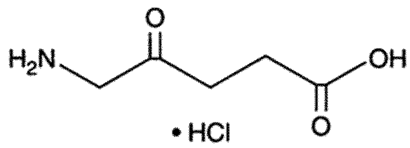

5-aminolevulinic acid hydrochloride included in the composition according to the present invention may be administered in an amount of 18 to 20 mg per patient's body weight, the 5-aminolevulinic acid hydrochloride solution may be orally administered, and, in the case of patient surgery, may be taken 2 to 4 hours before induction of anesthesia.

FIG. 1 is a photograph illustrating a patient's brain tumor area emitting a fluorescent color when a doctor performed surgery 6 hours after a patient drank the composition including 5-aminolevulinic acid hydrochloride according to the present invention.

That is, FIG. 1 illustrates a patient's brain tumor area emitting a fluorescent color when a doctor operates 6 hours after drinking 19 mg/kg of 5-aminolevulinic acid hydrochloride per patient's body weight (kg) 3 hours before induction of anesthesia in the case of patient surgery.

Referring to FIG. 1, a patient's brain tumor area emits a fluorescent color when a doctor performs an operation 6 hours after the patient drinks the composition including 5-aminolevulinic acid hydrochloride according to the present invention, so that a normal cell structure is damaged or destroyed and only the cell tissue in a tumor site can be safely removed.

In addition, the composition including 5-aminolevulinic acid hydrochloride according to the present invention may be prepared by dissolving 1.5 g of 5-aminolevulinic acid hydrochloride in 50 mL of purified water (pure water) so that 30 mg of 5-aminolevulinic acid hydrochloride may be contained per 1 mL of the prepared solution including 5-aminolevulinic acid hydrochloride.

The solution containing 5-aminolevulinic acid hydrochloride prepared as described above may be a transparent, colorless to slightly yellow liquid.

The composition including 5-aminolevulinic acid hydrochloride according to an embodiment of the technical idea of the present invention which is provided as an oral drug composition for fluorescence-induced malignant tissue visualization has been described as an example in which a patient orally administers and drinks. However, the oral drug composition for fluorescence-induced malignant tissue visualization including 5-aminolevulinic acid hydrochloride of the present invention may also be used in the form of a patch applied to the skin of a patient in addition to the oral administration form as described above, and the concentration of 5-aminolevulinic acid hydrochloride may be adjusted to less than 10% for cosmeceutical purposes.

A composition including 5-aminolevulinic acid hydrochloride according to another embodiment of the technical idea of the present invention may be a cosmetic composition for acne skin improvement.

That is, the composition including 5-aminolevulinic acid hydrochloride according to another embodiment of the technical idea of the present invention contains 5-aminolevulinic acid hydrochloride that is a material used in photodynamic therapy (PDT) and thus may be used as a cosmetic composition for acne skin improvement.

The composition including 5-aminolevulinic acid hydrochloride according to another embodiment of the technical idea of the present invention which is provided as a cosmetic composition for acne skin improvement includes 5-aminolevulinic acid hydrochloride, fermented black codonopsis lanceolata extract, cordyceps extract, krill oil, polydeoxyribonucleotide sodium, propylene glycol, hyaluronidase, L-carnitine, caffeine, origanum vulgare leaf oil, ascorbic acid (vitamin C), allantoin, cetyl alcohol and mineral water.

In addition, the cosmetic composition for acne skin improvement containing 5-aminolevulinic acid hydrochloride according to the present invention includes 8 to 12 % by weight of 5-aminolevulinic acid hydrochloride, 0.1 to 2.5 % by weight of fermented black codonopsis lanceolata extract, 1 to 3 % by weight of cordyceps extract, 0.5 to 1.5 % by weight of krill oil, 1 to 2 % by weight of polydeoxyribonucleotide sodium, 10 to 20 % by weight of propylene glycol, 1 to 3 % by weight of hyaluronidase, 3 to 5 % by weight of L-carnitine, 2 to 4 % by weight of caffeine, 0.1 to 1.0 % by weight of origanum vulgare leaf oil, 1 to 5 % by weight of ascorbic acid (vitamin C), 1 to 3 % by weight of allantoin, 5 to 10 % by weight of cetyl alcohol and the balance of mineral water.

The fermented black codonopsis lanceolata extract may be extracted from fermented black codonopsis lanceolata. The codonopsis lanceolata (Codonopsis lanceolata) is a perennial root plant belonging to the bellflower family and is wild in the mountainous regions of Korea, China and Japan. The codonopsis lanceolata has been called one of the five ginsengs along with ginseng since ancient times and has been widely used as food and herbal ingredients.

As main components of the codonopsis lanceolata, there are polyphenol, saponin, triterpene, a steroid-based compound, polyacetylene such as lobetyolin, and the like. The codonopsis lanceolata has been reported to have physiological activities such as neutral lipid and cholesterol suppression, antioxidant effect, antimutation effect and antitumor effect, and research is being conducted to use it as a material for healthy functional foods.

In addition, in oriental medicine, the codonopsis lanceolata has been used for disease treatment due to excellent effects thereof in swelling, tonic, antipyretic, expectorant, drainage, recovery after illness and promotion of lactation, and the like.

The fermented black codonopsis lanceolata extract may be prepared according to the following method.

First, codonopsis lanceolata is prepared and washed to remove foreign substances.

In the step, foreign substances attached to the codonopsis lanceolata may be removed by washing the prepared codonopsis lanceolata with purified water.

Next, the washed codonopsis lanceolata is dried to remove moisture remaining inside the codonopsis lanceolata.

In the step, the washed codonopsis lanceolata may be dried using a dryer or shade to prevent the moisture contained inside the codonopsis lanceolata from being rapidly evaporated and to prevent the loss of its own fragrance when drying in the sun. For example, the washed codonopsis lanceolata may be dried for 40 to 60 hours in a dryer maintained at 23 to 27.

That is, in the step, the moisture contained in the codonopsis lanceolata is gradually and evenly evaporated under the same process conditions as the above conditions so that the destruction of the cell tissue of the codonopsis lanceolata facilitates the penetration of air, thereby promoting fermentation.

Next, a fermentation broth for fermenting the dried codonopsis lanceolata may be prepared.

In the step, the fermentation broth may be prepared according to the following method.

To prepare the fermentation broth, first, nonglutinous rice may be prepared, washed, and then soaked in water.

In the step, the washed nonglutinous rice may be immersed at 25 to 30 °C for 50 to 100 minutes.

Next, the soaked nonglutinous rice may be heated to prepare hard-cooked rice.

In the step, hard-cooked rice suitable for culturing ferment bacillus may be prepared by mixing 1.5 to 2 times the weight of water with the soaked nonglutinous rice, and then heating it in a heat source at a temperature of 110 to 130 °C for 50 to 100 minutes.

Next, a fermentation broth may be prepared by mixing the hard-cooked rice with a ferment bacillus, yeast, minerals and purified water.

In the step, with respect to 100 parts by weight of the hard-cooked rice, 3 to 7 parts by weight of a ferment bacillus, 4 to 8 parts by weight of yeast, 0.5 to 1.5 parts by weight of minerals and 80 to 120 parts by weight of purified water in a weight ratio are mixed.

As the ferment bacillus, Aspergillus oryzae may be used. Aspergillus oryzae is a known strain used in the production of fermented soybeans and has excellent starch decomposition ability. The optimum temperature for growth of Aspergillus oryzae is usually around 30 °C, and it prefers high temperature and high humidity, and generates organic acids such as kojic acid and gluconic acid. In addition, Aspergillus oryzae is widely used as an excellent fermentation fungus that can produce soy sauce, soybean paste, red pepper paste, etc. by using the action of amylase, i.e., the power to decompose starch into sugar, and commercially known Aspergillus oryzae may be used.

The minerals refer to minerals that are absorbed into the codonopsis lanceolata during the fermentation and expansion process of the codonopsis lanceolata and that are necessary nutrients in relatively small amounts for the growth, maintenance and reproduction of the human body. As the minerals, at least one selected from the group consisting of calcium, phosphorus, potassium, magnesium, manganese, iodine, selenium, molybdenum, and cobalt may be used.

Next, fermented codonopsis lanceolata may be prepared by mixing the dried codonopsis lanceolata and the fermentation broth to ferment the codonopsis lanceolata.

In the step, 100 parts by weight of the dried codonopsis lanceolata may be mixed with 30 to 50 parts by weight of the fermentation broth, followed by fermenting at 24 to 26 °C for 20 to 40 hours. When the fermentation is performed below the lower value of the limit, the codonopsis lanceolata may be difficult to fully ferment, whereas, when the fermentation exceeds the upper value of the limit, the properties of the prepared fermented black codonopsis lanceolata may deteriorate.

Next, the fermented codonopsis lanceolata may be washed and cut, and the cut fermented codonopsis lanceolata may be dried to remove moisture remaining in the cut fermented codonopsis lanceolata.

In the step, the fermented codonopsis lanceolata may be cut to certain length units, and then dried in a dryer maintained at 30 to 35 °C for 30 to 40 hours.

Next, the cut and dried fermented codonopsis lanceolatea may be heated and steamed and then aged, thereby preparing fermented black codonopsis lanceolata.

In the step, with respect to the cut and dried fermented codonopsis lanceolata 100 parts by weight, a mixture of paraffin and cooking oil mixed in a weight ratio of 1:1 was fed in an amount of 2000 to 3000 parts by weight into a heating container, and then heated at 90 to 95°C for 10 to 20 minutes, followed by steaming while maintaining a temperature of 80 to 85°C for 40 to 50 minutes and aging at 75 to 80°C for 5 to 10 minutes. As a result, one steaming and aging process was performed. This process was repeated 10 times, thereby preparing fermented black codonopsis lanceolata.

In the step, the paraffin may be liquid paraffin. The liquid paraffin is a hydrocarbon-based mineral oil with non-drying properties. For example, NF or FDA grade white mineral oil having a molecular weight in a range of C8 to C32 may be used. Here, the standard of kinematic viscosity is ISO VG (viscosity grade) and may be in a range of 6 to 100 CST at 37.8°C.

In addition, the cooking oil is a fatty acid ester (Glyceride) of glycerol obtained from animal or plant seeds, nuts, fruits, etc., and refers to an edible oil added as a seasoning to give a flavor when cooking food or widely used for frying or roasting various types of fish or meat.

Next, a fermented black codonopsis lanceolata extract may be extracted from the fermented black codonopsis lanceolata.

In the step, the fermented black codonopsis lanceolata may be extracted using a known extraction method. For the convenience of description and clarity of the technical idea of the present invention, a detailed description of the extraction of the fermented black codonopsis lanceolata is omitted.

The cordyceps extract may be prepared by extracting useful components of cordyceps. Cordycpin, a physiologically active substance, is known for its efficacy on the cordyceps. It has been found in clinical trials that cordycpin, which is a natural compound of the 3'-deoxyadenosine family, has antibacterial, antitumor and anticancer effects.

In particular, Cordyceps militaris contains a relatively large amount of cordycpin among several cordyceps, and its fruiting body is known to protect the lungs and strengthen the kidneys. In addition, Cordyceps militaris is widely recognized for its treatment of tuberculosis, jaundice, etc., and its effectiveness as a tonic agent, and is also used as an expensive herbal medicine mainly used for post-morbid body control and restorative medicine.

In addition, pharmacological components of Cordyceps militaris contain properties that enhance immune function, so it is effective for colds, pulmonary tuberculosis, chronic cough, asthma, seizures, anemia, male sexual dysfunction, high blood pressure, and the like. In addition, Cordyceps militaris contains a large amount of polysaccharides together with D-mannitol and quinic acid in addition to cordycpin, and is known to have a high content of various amino acids.

The cordyceps extract may be prepared according to the following method.

First, cordyceps are washed to remove foreign substances adhering to the surface of the cordyceps.

In the step, the cordyceps may be washed with purified water after immersing the cordyceps in a mixture of lemon juice and salt solution for 3 to 5 minutes. Here, the lemon juice and the salt solution may be mixed at a weight ratio of 1:4.

The lemon juice and salt solution serve to sterilize the cordyceps and at the same time prevent it from easily decaying. A salt solution at a concentration of 1 to 3% by weight may be used.

Next, the washed cordyceps may be vacuum-dried by irradiating microwaves in a reduced pressure atmosphere.

In the step, the step may be performed by irradiating the washed cordyceps with microwaves under a pressure of 60 to 70 mbar. This step may be performed by irradiating microwaves of 2.2 GHz onto the washed cordyceps at 40 to 50°C for 5 to 10 minutes.

The microwaves exhibit a volumetric heating effect of penetrating into and heating the inside of cordyceps. When the microwaves are irradiated, water molecules inside the cordyceps vibrate or rotate according to the polarity conversion of the microwaves, and such polarization vibrations may lead to friction between molecules and cause an exothermic phenomenon. When cordyceps are dried by irradiating microwaves under reduced pressure, swelling occurs due to heating inside the cordyceps, and a sterilization effect occurs as the cell membrane inside the cordyceps is destroyed by this instant internal heating and swelling. In additon, the active ingredients of cordyceps can be effectively leached during subsequent extraction by swelling due to internal heating of cordyceps.

Next, the vacuum-dried cordyceps may be aged. In the step, the properties of the cordyceps may be stabilized by storing the vacuum-dried cordyceps at 10 to 15 °C for 30 to 40 hours.

Next, the aged cordyceps may be mixed with purified water and then heated to produce a cordyceps extract enriched with useful components of cordyceps.

In the step, 1000 to 1500 g of cordyceps in a weight ratio may be mixed per 10 to 12 liters (L) of purified water, followed by first heating at 90 to 95 °C for 60 to 80 minutes and secondarily heating at 70 to 75°C for 300 to 400 minutes.

When the step is performed below the lower limit of the range, there may be a problem in that useful components of cordyceps are not sufficiently extracted. When the step is performed beyond the upper limit of the range, cordyceps residues may occur, resulting in deterioration of the properties of the cordyceps extract.
the krill oil may be prepared using krill, and the krill is known to contain a phospholipid compound with a structure similar to that of human cell membrane components. Therefore, it is known that oil extracted from krill is better absorbed into the human body than other fish oils.

In addition, kill oil contains vitamins A, E, and D in addition to lipids such as omega-3, and especially contains a substance called astaxanthin, which is a powerful antioxidant. Accordingly, it has been reported that the antioxidant function of krill oil reaches 48 times that of general fish oil.

That is, krill is nutritionally composed of high-quality protein and lipid, fresh krill contains up to 10% lipid, and the lipid of krill is eicosapentaenoic acid (EPA), which is a polyunsaturated fatty acid omega-3, and docosahexaenoic acid (DHA) in a content of about 40%, and phospholipids in a content of about 40 to 50%. In addition, it is useful because it contains astaxanthin, an antioxidant of fat-soluble carotenoids.

To prepare the krill oil, first, krill may be prepared and washed, and then dried.

Drying of the washed krill may include a first drying and a second drying. The first drying may be performed by drying the washed krill at 50 to 60°C for 5 to 15 hours to first remove moisture remaining in the washed krill.

In addition, the second drying may be performed by drying the first dried krill with hot air. The second drying may be performed by applying hot air at 110 to 130°C to the first dried krill for 30 to 50 minutes.

Next, the second dried krill may be pulverized and powdered, and then aged to prepare an aged krill powder.

In the step, the second dried krill may be powered by pulverizing the second dried krill to a particular size of 5 to 10 mm, and the powered krill may be aged by storing the powered krill at 6 to 8°C for 20 to 40 hours.

Next, the aged krill powder is mixed with an enzyme and an acid, and then stored to be enzymatically decomposed.

That is, in the step, the aged krill powder may be mixed with an enzyme and an acid, and then the aged krill powder mixed with the enzyme and the acid is stored at 36 to 38°C for 10 to 30 hours, thereby being enzymatically decomposed. To enzymatically decompose the aged krill powder, an enzyme capable of separating proteins, chitin, and immunoactive polysaccharides present in the krill may be used.

In the step, commercially available known protease, chitinase, pectinase and xylanase may be used as the enzyme. As the acid, citric acid and ascorbic acid may be used. Specifically, pH of the aged krill powder mixed with the enzyme and the acid may be adjusted to 3.5 to 6.5 by mixing 0.01 to 0.03 parts by weight of the protease, 0.03 to 0.05 parts by weight of chitinase, 0.005 to 0.009 parts by weight of pectinase, 0.008 to 0.012 parts by weight of xylanase, 0.01 to 0.1 parts by weight of citric acid and 0.01 to 0.1 parts by weight of ascorbic acid with respect to 100 parts by weight of the aged krill powder based on a weight ratio.

Next, oil is separated from the enzymatically decomposed aged krill powder, thereby preparing krill oil.

A method of extracting oil from the enzymatically decomposed aged krill powder in the step is a known technique to those of ordinary skill in this technical field. For convenience of description and clarity of the technical idea of the present invention, a detailed description thereof is omitted.

The polydeoxyribonucleotide sodium can relieve inflammation of damaged tissue and promote cell regeneration. For example, Polydeoxyribonucleotide sodium can interact with adenosine receptors to promote the expression of vascular endothelial growth factor, thereby promoting wound healing, promoting epithelial cell growth, promoting extracellular matrix maturation, and increasing microvascular density to promote angiogenesis.

Propylene glycol maintains the formulation stability of a cosmetic composition by preventing the formulation of the cosmetic composition from changing or decomposing, has antibacterial action, inhibits the growth of bacteria, and can act as a wetting agent, a moisturizing agent, and a preservative.

The aminophylline functions as a Phospho Di Esterase (PDE) inhibitor and may be used to promote lipid degradation.

The hyaluronidase is excreted through the lymphatic fluid after the destruction of fat cells, and when used together with aminophylline, it can help remove edema and improve circulation by controlling the release of aminophylline.

The L-carnitine combines with fatty acids to be transported and utilized as energy, and fatty acids can inhibit feedback, can secrete ATP to increase lipolytic enzyme activity, and can cause dehydration of neutral fat.

The caffeine can function as a Phospho Di Esterase inhibitor and promote lipolysis.

The origanum vulgare leaf oil can be manufactured using oregano leaves. The oregano (Origanum vulgare) is

native to the Mediterranean coast, was used for medicinal purposes in ancient Greece and was used in cooking in ancient Rome. Oregano is harmless and beneficial to the human body, so still used as a natural preservative for meat storage and as a spice in Italian cuisine.

In addition, oregano, also known as a natural antibiotic, contains a lot of phenolic acid and flavonoids, so it has high antioxidant activity. Oregano is said to be effective for tonic, diuretic, stomach, appetite enhancement, soothing, sterilization, headache, motion sickness, and insomnia, so it is mainly boiled as a tea or used as a bathing agent.

The ascorbic acid (vitamin C) is involved in the synthesis of collagen, a major protein constituting connective tissue such as skin, skeleton, blood vessels, cartilage, etc., and is used as a coenzyme for various enzyme reactions in vivo. In addition, the ascorbic acid (vitamin C) converts iron into a reduced form in the small intestine, thereby increasing the absorption of vegetable iron that is not bound to hemoglobin and preventing inflammation or aging.

The allantoin helps with skin soothing effect and cell activation, the cetyl alcohol is used as a water-soluble thickener, and the mineral water may function as a solvent for dispersing and dissolving the composition.

Hereinafter, an example of a cosmetic composition for acne skin improvement including 5-aminolevulinic acid hydrochloride according to another embodiment of the technical idea of the present invention and a comparative example thereof are described in detail with reference to the accompanying drawings.

<Example>

10 % by weight of 5-aminolevulinic acid hydrochloride, 1.5 % by weight of a fermented black codonopsis lanceolata extract, 2 % by weight of a cordyceps extract, 1 % by weight of krill oil, 1.5 % by weight of polydeoxyribonucleotide sodium, 15 % by weight of propylene glycol, 2 % by weight of hyaluronidase, 4 % by weight of L-carnitine, 3 % by weight of caffeine, 0.5 % by weight of origanum vulgare leaf oil, 3 % by weight of ascorbic acid (vitamin C), 2 % by weight of allantoin, 8 % by weight of cetyl alcohol and the balance of mineral water were mixed to prepare a cosmetic composition for acne skin improvement.

<Comparative example>

A commercially available acne ointment (manufactured by P company) was prepared and used as a cosmetic composition for acne skin improvement of a comparative example.

## 1. Antibacterial activity evaluation experiments

The following experiments were conducted to evaluate the antimicrobial activity of cosmetic compositions for acne skin improvement according to an example and a comparative example. In the antimicrobial activity evaluation experiments, acne bacteria, propionibacterium acnes (ATCC 6919), was used as test bacteria.

Acne bacteria were inoculated into BHI broth and pre-cultured, and then inoculated at a concentration of 1% into 100 ml of a new sterilized BHI broth containing 5% of each composition, followed by culturing in an anaerobic incubator for 2 days, and then the number of bacteria was measured.

[Table 1]

| Classification | Acne bacteria (P.acnes) | |
|---|---|---|
| | Concentration (cfu/ml) of inoculated bacteria | Bacteria concentration (cfu/ml) after culturing |
| Example | $8.75 \times 10^5$ | $3.35 \times 10^2$ |
| Comparative example | $8.75 \times 10^5$ | $7.42\ 10^4$ |

Referring to the above [Table 1], it can be confirmed that the cosmetic composition for acne skin improvement prepared according to the example shows greatly improved antimicrobial activity against acne bacteria.

## 2. Antioxidative activity experiments

Radical scavenging activity was used using the cosmetic composition for acne skin improvement according to the example as a sample.

The antioxidative activity (electron donation ability, EDA%) of a sample was measured by modifying the Blois method. 2 mL of the sample was mixed with 2 mL of a 0.2 mM DPPH(1,1-diphenyl-2-picryl hydrazyl) solution, and then allowed to stand for 30 minutes, followed by measuring an absorbance at 517 nm.

The radical scavenging activity was expressed as the rate of decrease in absorbance between the sample-added group and the non-added group. The radical scavenging activity was calculated according to the following equation, and results are summarized in [Table 2] below. As a sample for the control, BHT (synthetic antioxidant) was used.

Radical scavenging activity (%) = (1 - Absorbance of sample-added group/Absorbance of non-added group)×100

[Table 2]

| Classification | Radical scavenging activity |
|---|---|
| Example | 45.4 ± 0.65 |
| Control | 55.3 ± 0.45 |

Referring to [Table 2], the cosmetic composition for acne skin improvement prepared according to the example was confirmed to have excellent antioxidative activity, and to have better radical scavenging activity than a conventional synthetic antioxidant used as a control.

Therefore, the cosmetic composition for acne skin improvement prepared according to the example has an excellent antioxidant effect of improving acne skin against active oxygen and free radicals, and is expected to greatly enhance the physiological activity of acne skin.

3. Sebum excretion inhibitory efficacy evaluation

The sebum excretion inhibitory effects of the cosmetic compositions for acne skin improvement prepared according to the example and the comparative example were analyzed.

To evaluate the sebum excretion inhibitory effect, 30 women and 30 men with oily skin aged 15 to 25 were divided into 3 groups of 10 each, and the cosmetic compositions for acne skin improvement prepared according to the example and the comparative example were respectively applied to the face twice a day in the morning and evening. When 5 days elapsed, the amount of sebum secretion on the face was measured using a skin moisture meter.

The experiments were carried out under constant temperature and humidity conditions, i.e., 23°C and a relative humidity of 42%, and the amount of sebum excretion was taken as a default value. The cosmetic compositions for acne skin improvement according to the example and the comparative example were applied, and then the sebum inhibitory effect was evaluated by measuring a change in the amount of sebum excretion, and a sebum secretion amount changed from an initial value was calculated using [Equation 1] below. Results (average value of each group) are shown in [Table 3] and FIG. 1 (St=0 represents the initial sebum secretion amount before application, St=i represents the sebum secretion amount 5 days after application).

As a control, the sebum excretion inhibitory effect of a subject who was not applied with the cosmetic composition for acne skin improvement was analyzed.

[Equation 1]

Sebum excretion amount = (St-0) - St-1)/ St-0 X 100

[Table 3]

| Classification | Sebum secretion inhibition rate (%) after 5 days |
|---|---|
| Example | 72 |
| Comparative example | 53 |
| Control | 3 |

FIG. 2 is a graph illustrating a change in the amount of sebum excretion after 5 days of applying the cosmetic compositions for acne skin improvement according to the example and the comparative example to the subject's acne

skin.

Referring to [Table 3] and FIG. 2, it can be confirmed that the cosmetic composition for acne skin improvement according to the example exhibits excellent sebum excretion inhibitory effect, compared to the cosmetic composition for acne skin improvement.

4. Inflammation-reducing effect measurement

Inflammation-reducing effect of each of the cosmetic compositions for acne skin improvement prepared according to the example and the comparative example was measured. First, macrophages (Raw 2647 mouse cell) were dispensed at a concentration of 4 × 10 5 cells per well of a 96-well microplate, and then treated with the composition of each of the example and the comparative example at a concentration of 1,000 μg/mℓ under conditions of 35°C and 5% CO2 for 20 hours. After the treatment, each supernatant was taken and the amount of TNF-α was measured by the sandwich ELISA. Results are summarized in [Table 4] below.

[Table 4]

| Classification | TNF-α assay | |
|---|---|---|
| | Example | Comparative example |
| TNF-α expression rate (%) | 62.5 | 43.2 |

Referring to [Table 4], it can be confirmed that the cosmetic composition for acne skin improvement according to the example exhibits high TNF-α induction effect compared to the comparative example. In addition, it can be confirmed that the cosmetic composition for acne skin improvement according to the example induces TNF-α production to increase the activity of macrophages, thereby exhibiting excellent immune-enhancing effect.

5. Observation of acne improvement

The cosmetic composition prepared according to the example was applied to the subject's acne skin, and then an experiment was carried out using photodynamic therapy (PDT). After a predetermined time elapses, the subject's acne skin condition was observed.

FIGS. 3A, 4A, 5A, and 6A are photographs showing the subject's acne skin condition before applying the cosmetic composition prepared according to the example, and FIGS. 3B, 4B, 5B, and 6B are photographs showing the subject's acne skin condition after applying the cosmetic composition prepared according to the example once a day for 20 days.

Referring to FIGS. 3A to 6B, it can be confirmed that the subject's acne skin condition (FIGS. 3B, 4B, 5B, and 6B) after applying the cosmetic composition prepared according to the example was improved compared to the subject's acne skin condition (FIGS. 3A, 4A, 5A, and 6A) before applying the cosmetic composition prepared according to the example.

Hereinafter, an example of photodynamic therapy using the composition including 5-aminolevulinic acid hydrochloride according to the present invention with reference to FIGS. 7A and 7B is described in detail.

Although oral administration of the composition for photodynamic therapy according to the present invention is described as an example in the following example, a method of taking the composition for photodynamic therapy according to the technical idea of the present invention is not limited to the oral administration.

That is, the composition for photodynamic therapy including 5-aminolevulinic acid hydrochloride according to the present invention may be prepared in various forms and administered to a patient. For example, the composition for photodynamic therapy including 5-aminolevulinic acid hydrochloride according to the present invention may be prepared and used in the form of a drug for internal or external use.

Specifically, the drug for internal may be prepared and administered in various known forms such as tablets (pills), powders, granules, solutions (potions), syrups, and drinks, and the drug for external use may be prepared and administered in various known forms such as patches (pastes), ointments, creams, gels, liquids, and injections.

The composition for photodynamic therapy including 5-aminolevulinic acid hydrochloride according to the present invention includes 5-aminolevulinic acid hydrochloride, indole-3-acetic acid (IAA), polyethyleneimine, self-assembled ligand particles, purified water, a carrier, glycerin fatty acid ester and an emulsifier.

In addition, the composition for photodynamic therapy including 5-aminolevulinic acid hydrochloride according to the present invention may include 30 to 50 parts by weight of 5-aminolevulinic acid hydrochloride, 10 to 20 parts by weight of indole-3-acetic acid (IAA), 1 to 5 parts by weight of polyethyleneimine, 5 to 15 parts by weight of self-assembled ligand particles, 10 to 20 parts by weight of purified water, 10 to 20 parts by weight of a carrier, 20 to 30 parts by weight

of glycerin fatty acid ester, and 10 to 20 parts by weight of an emulsifier based on a weight ratio.

In addition, after 6 hours of drinking and administering the composition for photodynamic therapy according to the present invention including 5-aminolevulinic acid hydrochloride, indole-3-acetic acid (IAA), polyethyleneimine, self-assembled ligand particles, a carrier, glycerin fatty acid ester and an emulsifier according to the patient's weight, light in the wavelength range of 660 to 680 nm was irradiated onto the patient's tumor site and photoactivated in vivo, thereby making the patient's tumor site emit a fluorescent or phosphorescent color.

Specifically, if the composition for photodynamic therapy including 5-aminolevulinic acid hydrochloride according to the present invention is prepared in the form of a potion, glioblastoma surgery may be performed 6 hours after taking a bottle of 10 cc potion when the patient's weight is less than 60 kg, and glioblastoma surgery may be performed 6 hours after taking a bottle of 10 cc potion twice, i.e., taking 2 bottles of potion, when the patient's weight exceeds 60 kg.

The 5-aminolevulinic acid hydrochloride makes the tumor area emit a fluorescent color after a patient drinks it, so that the tumor area can be removed while protecting normal tissue as much as possible during surgery.

That is, the 5-aminolevulinic acid hydrochloride is a material that can be used for photodynamic therapy (PDT). The photodynamic therapy (PDT) is a technology for treating incurable diseases such as cancer without surgery by using a photo-sensitizer (hereinafter referred to as "photosensitizer").

The photodynamic therapy (PDT) has been actively researched since the early 20th century, and is currently used in the diagnosis and treatment of cancer, allogeneic and autologous bone marrow transplantation, resolution of host-sponsored transplantation reactions by photopheresis, antibiotics, treatment of infectious diseases such as AIDS, organ and skin transplantation, treatment of arthritis, treatment of spinal muscular atrophy, and the like.

In addition, the photodynamic therapy (PDT) is used to strengthen immunity or to control and regulate immune responses, and the application range thereof is gradually expanding to regenerative medicine, biosensors, optogenetics, and the like.

In particular, PDT, which is used for cancer treatment, generates singlet oxygen or free radicals through a chemical reaction between abundant oxygen in the body and external light when a photosensitizer that is sensitive to light is injected into the body and irradiated with light from the outside. PDT is a treatment using the principle that the singlet oxygen or the free radical induces and destroys apoptosis of various lesions or cancer cells.

As photosensitizers currently used in PDT therapy, a porphyrin derivative, chlorin, bacteriochlorin, phthalocyanine, a 5-aminolevulinic acid derivative, porfimer sodium, and the like are known. The composition for photodynamic therapy according to the present invention may include 5-aminolevulinic acid hydrochloride.

The 5-aminolevulinic acid hydrochloride is a C-5 aliphatic compound represented by [Formula 2] below and is one of the intermediates found in the process of tetrapyrrole biosynthesis (prophyrin, chlorophyll, heme, vitamin B 12, etc.) of various organisms.

[Formula2]

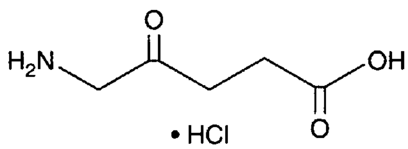

The indole-3-acetic acid (IAA) functions as a photosensitizer and has the property of being activated by light. The indole-3-acetic acid (IAA) may be used simultaneously with the 5-aminolevulinic acid hydrochloride and photoactivated by ultraviolet or visible light, thereby making the patient's tumor site emit a fluorescent color or a phosphorescent color.

The indole-3-acetic acid (IAA) may be represented by [Formula 3] below:

[Formula3]

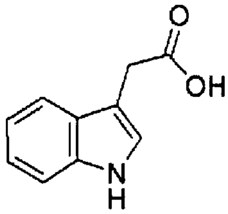

The polyethyleneimine may be used as a biocompatible polymer. The polyethyleneimine exhibits excellent stability in vivo when orally administered and continues to accumulate at a tumor site for a sufficient period of time due to a high biodistribution in the blood, thereby making a patient's tumor site emit a fluorescent color or a phosphorescent color.

The polyethyleneimine may be widely used as a gene carrier, and is very soluble in water-soluble solvents due to many amine groups therein. Polyethyleneimine is well known, and a commercially available polyethyleneimine may be used in the present invention.

The self-assembled ligand particles are ligand particles that specifically react to tumor cells and may be represented by [Formula 4] below.

[Formula4]

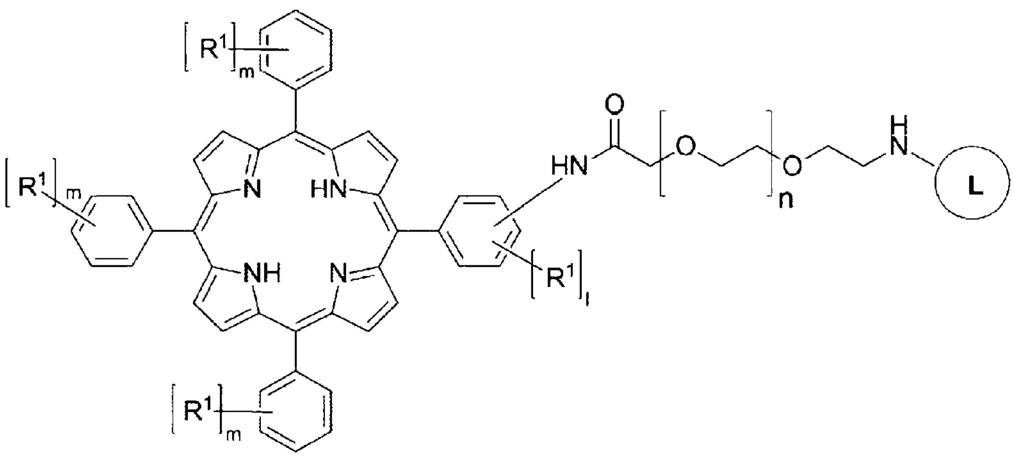

In [Formula 4], R1 is independently hydrogen, halogen or straight-chain or branched C1-10 alkyl, l is an integer of 2 or 3, m is an integer of 4 to 7, n is an integer of 1000 to 10,000, and

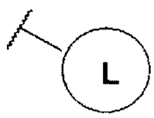

is a ligand that specifically reacts to tumor cells and may be any one selected from biotin or folic acid.

The self-assembled ligand particles refer to ligands that can bind to tumor cells, and the self-assembled ligand particles enable the composition for photodynamic therapy according to the present invention to selectively and specifically bind to and act on a patient's tumor site.

As the carrier, a substance that chemically combines a lipid and α-lipoic acid may be used. A photosensitizer generates reactive oxygen species according to light irradiation, and the carrier releases the composition from a patient's tumor site while α-lipoic acid scavenges the generated reactive oxygen species, so that the components of the composition for photodynamic therapy can be stably delivered to the patient's tumor site.

A lipid constituting the carrier may be one or more selected from the group consisting of L-α-phosphatidylcholine, 1,2-dipalmitoyl-sn-glycero-3-phosphoethanolamine) and 1,2-Distearoyl-sn-glycero-3-phosphoethanolamine).

The α-lipoic acid (LA) is an essential component of mitochondria, exists in various ways in animals and plants,

and mainly acts as a coenzyme in an enzyme reaction in mitochondria. LA is biosynthesized in all cells, and it is known that octanoic acid becomes a precursor of the C-8 fatty acid chain of lipoic acid and cysteine serves as a source of sulfur, resulting in the biosynthesis of lipoic acid.

The lipoic acid and dihydrolipoic acid, which is a reduced form of lipoic acid, are well known as antioxidants, protect vitamins C and E and glutathione to enhance effectiveness thereof, and suppress NF-kB to inhibit tissue damage caused by ultraviolet rays as an anti-inflammatory agent. This lipoic acid has been used as an important drug for various diseases in the pharmaceutical field for a long time.

The glycerin fatty acid ester may be one or more selected from the group consisting of triacetin, tributyrin, tricaproin, tricaprylin, tricaprine and triolein.

The emulsifier may be one or more selected from the group consisting of sorbitan ester (Span), polyoxyethylene sorbitan (Tween) and polyoxyethylene ether (Brij).

Hereinafter, an example of the composition for photodynamic therapy including 5-aminolevulinic acid hydrochloride according to the present invention is described in more detail with reference to the accompanying drawings.

<Example>

A composition for photodynamic therapy was prepared by mixing 40 parts by weight of 5-aminolevulinic acid hydrochloride, 15 parts by weight of indole-3-acetic acid (IAA), 3 parts by weight of polyethyleneimine, 10 parts by weight of self-assembled ligand particles, 15 parts by weight of purified water, 15 parts by weight of a carrier formed by chemically combining a lipid and $\alpha$-lipoic acid, 25 parts by weight of glycerin fatty acid ester (triacetin) and 15 parts by weight of an emulsifier (sorbitan ester (Span)) based on a weight ratio.

The composition for photodynamic therapy according to the example was administered by drinking in an amount of 35 mg/kg per patient's body weight (kg) and, after 6 hours, light in the 670 nm wavelength range was irradiated to the patient's tumor site and photoactivated in vivo.

FIG. 7A is a photograph illustrating the location of a patient's brain tumor before the patient drank the composition including 5-aminolevulinic acid hydrochloride according to the present invention, and FIG. 7B is a photograph illustrating a state in which a patient's brain tumor area emits a fluorescent color when a doctor performs an operation 6 hours after the patient drank the composition including 5-aminolevulinic acid hydrochloride according to the present invention.

Referring to FIGS. 7A and 7B, it can be confirmed that since a patient's brain tumor area emits a fluorescent color when a doctor performs an operation 6 hours after the patient drinks the composition including 5-aminolevulinic acid hydrochloride according to the present invention, only the cell tissue of the tumor site can be safely removed without damaging or damaging the normal cell structure.

Although embodiments of the present invention have been described with reference to the accompanying drawings, those skilled in the art will understand that the present invention can be easily changed or modified into other specified forms without change or modification of the technical spirit or essential characteristics of the present invention. Therefore, it should be understood that the aforementioned examples are only provided by way of example and not provided to limit the present invention. It should be understood that the scope of the present invention is defined by the following claims, rather than the detailed description, and the invention is to cover all modifications, equivalents, and alternatives falling within the spirit and scope of the invention as defined by the claims.

[Mode for Carrying Out the Invention]

A mode for carrying out the invention has been described together in the best mode for carrying out the invention above.

[Industrial Applicability]

The present invention can be used in various industrial fields related to a composition including 5-aminolevulinic acid hydrochloride.

## Claims

1. A composition comprising 5-aminolevulinic acid hydrochloride, the composition comprising 5-aminolevulinic acid hydrochloride, fermented black codonopsis lanceolata extract, cordyceps extract, krill oil, polydeoxyribonucleotide sodium, propylene glycol, hyaluronidase, L-carnitine, caffeine, origanum vulgare leaf oil, ascorbic acid (vitamin C), allantoin, cetyl alcohol and mineral water.

2. The composition according to claim 1, consisting: 8 to 12 % by weight of 5-aminolevulinic acid hydrochloride, 0.1 to 2.5 % by weight of fermented black codonopsis lanceolata extract, 1 to 3 % by weight of cordyceps extract, 0.5 to 1.5 % by weight of krill oil, 1 to 2 % by weight of polydeoxyribonucleotide sodium, 10 to 20 % by weight of propylene glycol, 1 to 3 % by weight of hyaluronidase, 3 to 5 % by weight of L-carnitine, 2 to 4 % by weight of caffeine, 0.1 to 1.0 % by weight of origanum vulgare leaf oil, 1 to 5 % by weight of ascorbic acid (vitamin C), 1 to 3 % by weight of allantoin, 5 to 10 % by weight of cetyl alcohol and a balance of mineral water.

3. A composition comprising 5-aminolevulinic acid hydrochloride, the composition comprising 5-aminolevulinic acid hydrochloride, indole-3-acetic acid (IAA), polyethyleneimine, self-assembled ligand particles, purified water, a carrier, glycerin fatty acid ester and an emulsifier.

4. The composition according to claim 3, consisting 30 to 50 parts by weight of 5-aminolevulinic acid hydrochloride, 10 to 20 parts by weight of indole-3-acetic acid (IAA), 1 to 5 parts by weight of polyethyleneimine, 5 to 15 parts by weight of self-assembled ligand particles, 10 to 20 parts by weight of purified water, 10 to 20 parts by weight of a carrier, 20 to 30 parts by weight of glycerin fatty acid ester and 10 to 20 parts by weight of an emulsifier based on a weight ratio.

5. The composition according to claim 4, wherein the composition for photodynamic therapy comprising 5-aminolevulinic acid hydrochloride, indole-3-acetic acid (IAA), polyethyleneimine, self-assembled ligand particles, a carrier, glycerin fatty acid ester and an emulsifier is administered by drinking in an amount of 35 mg/kg per patient's body weight (kg), and after 6 hours, light in a wavelength range of 660 to 680 nm is irradiated to a tumor site of a patient to be photoactivated in vivo.

FIG. 1

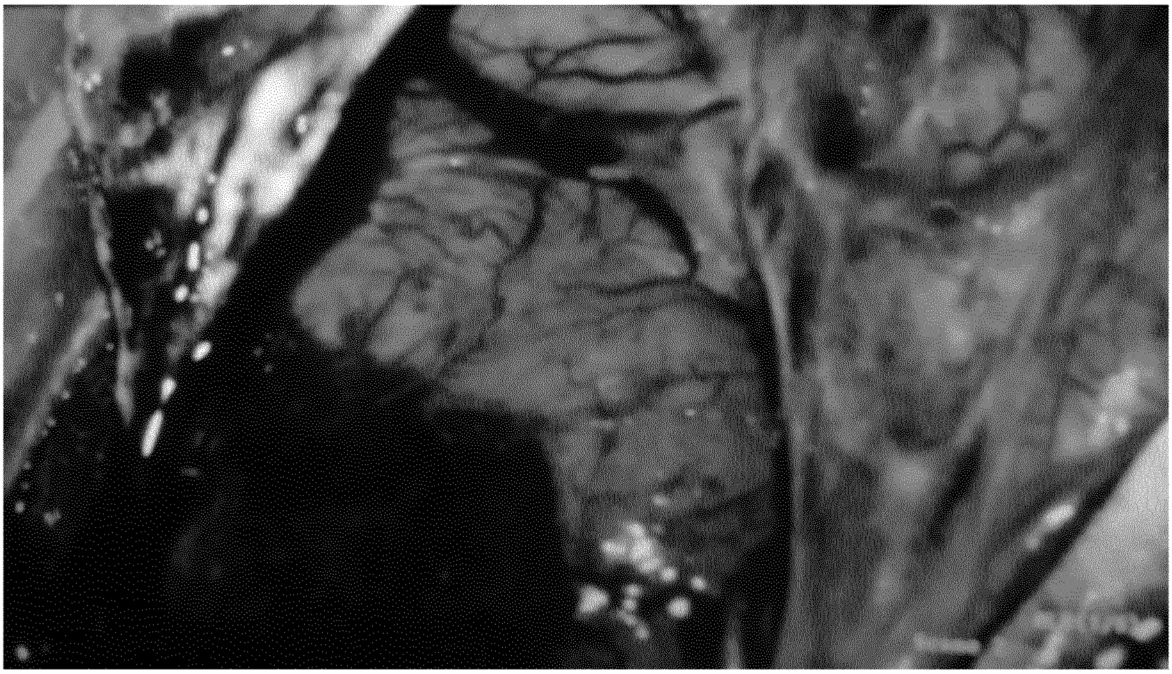

FIG. 2

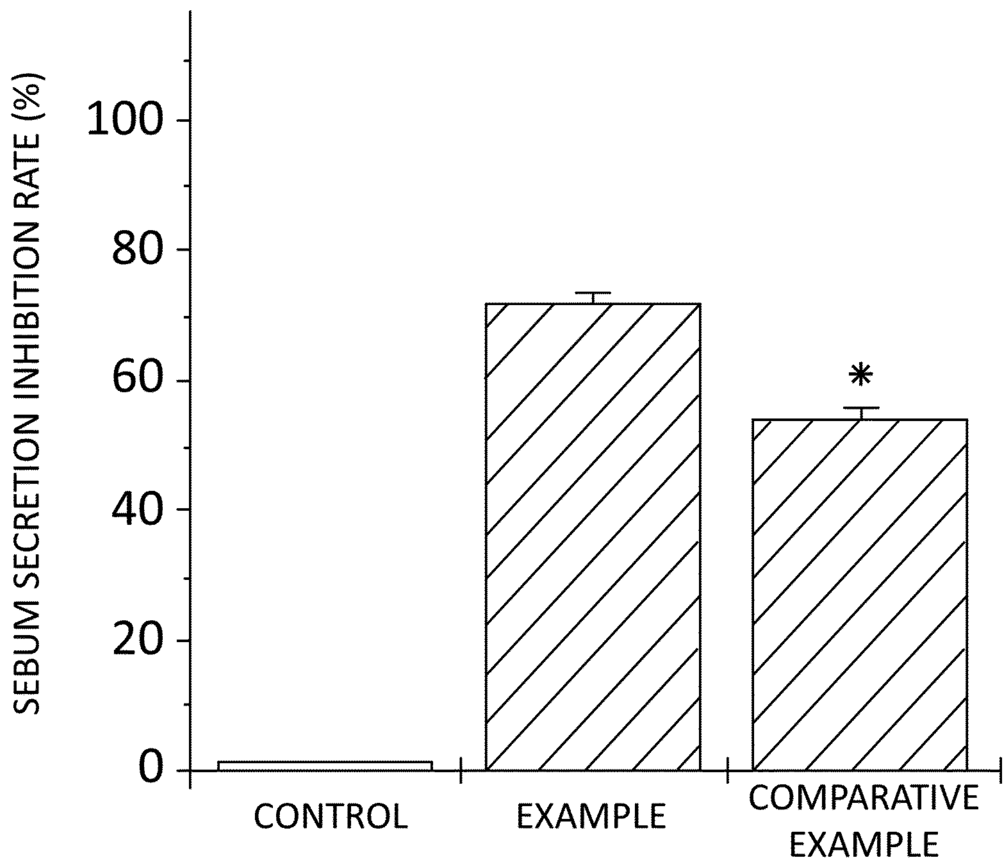
SEBUM SECRETION INHIBITION RATE (%)
100
80
60
40
20
0
*
CONTROL
EXAMPLE
COMPARATIVE EXAMPLE

FIG. 3A

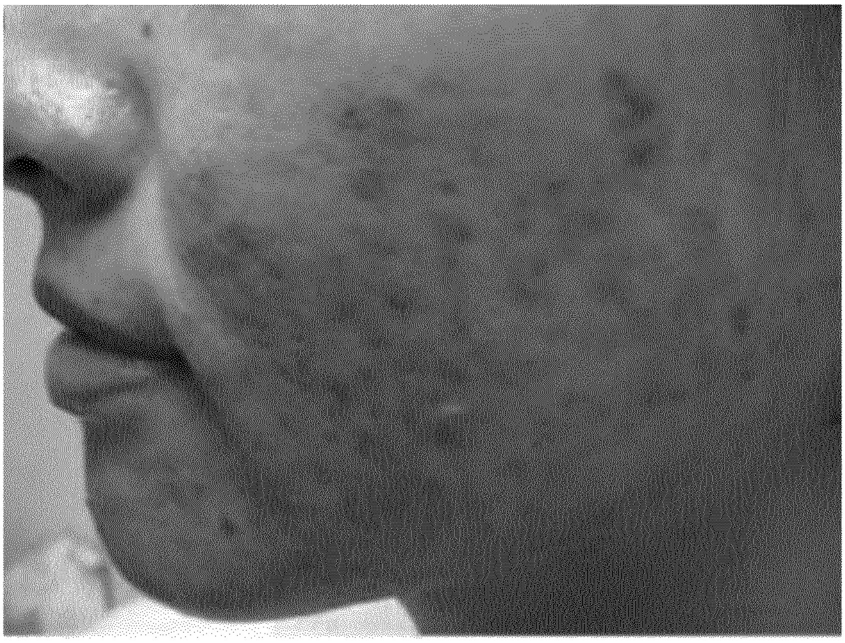

FIG. 3B

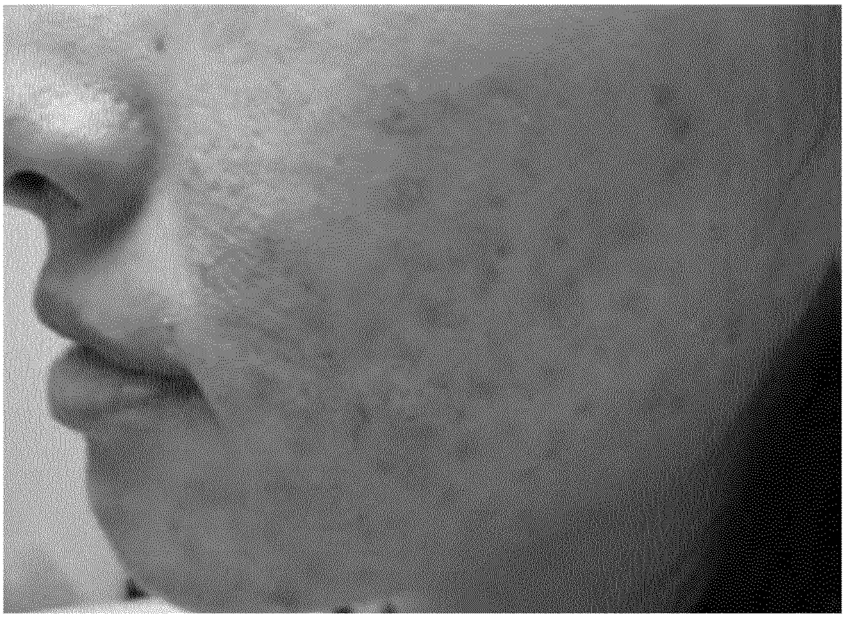

FIG. 4A

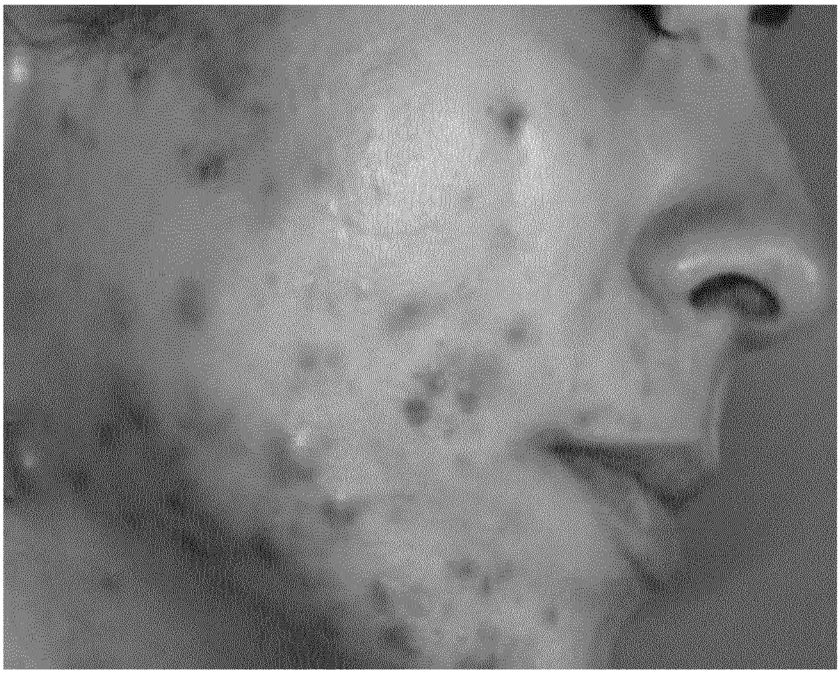

FIG. 4B

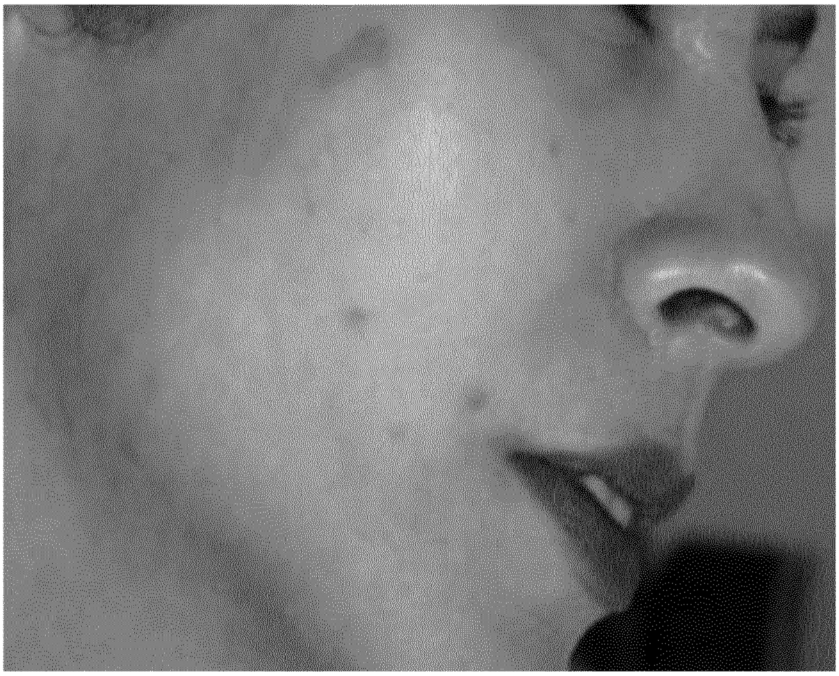

FIG. 5A

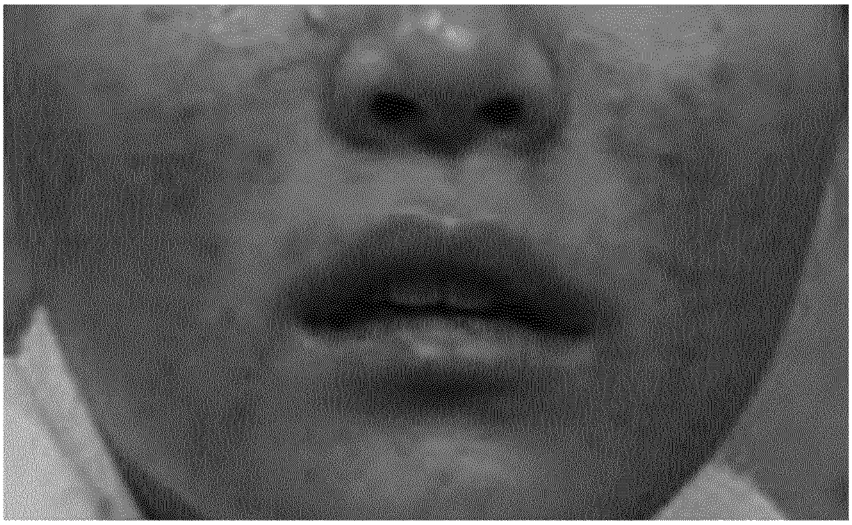

FIG. 5B

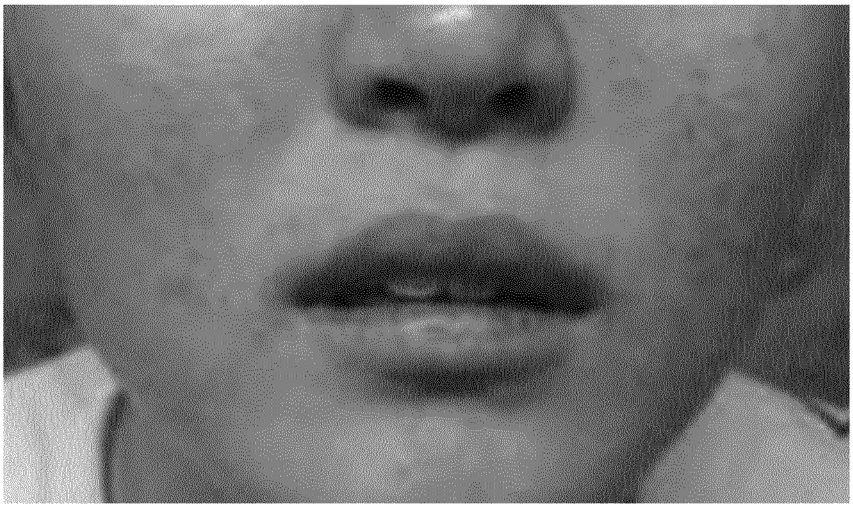

FIG. 6A

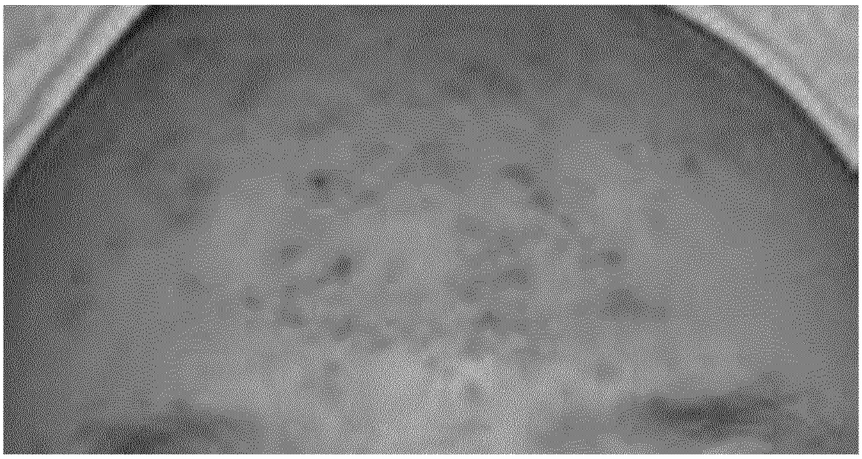

FIG. 6B

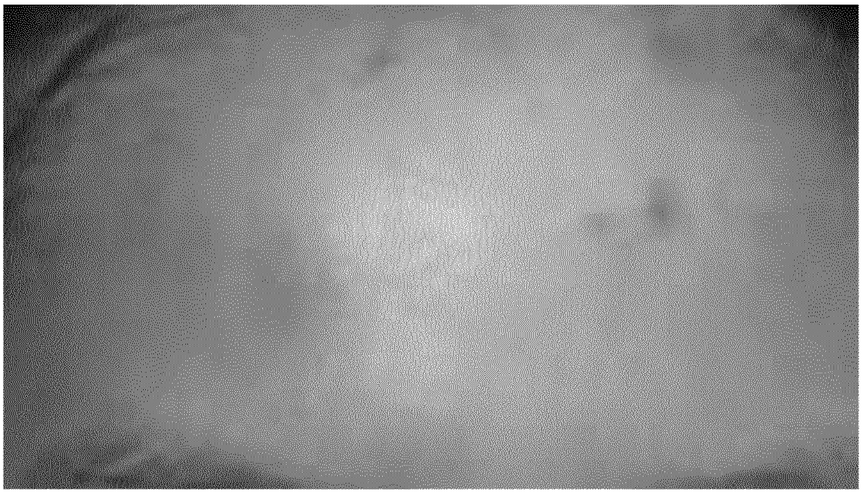

FIG. 7A

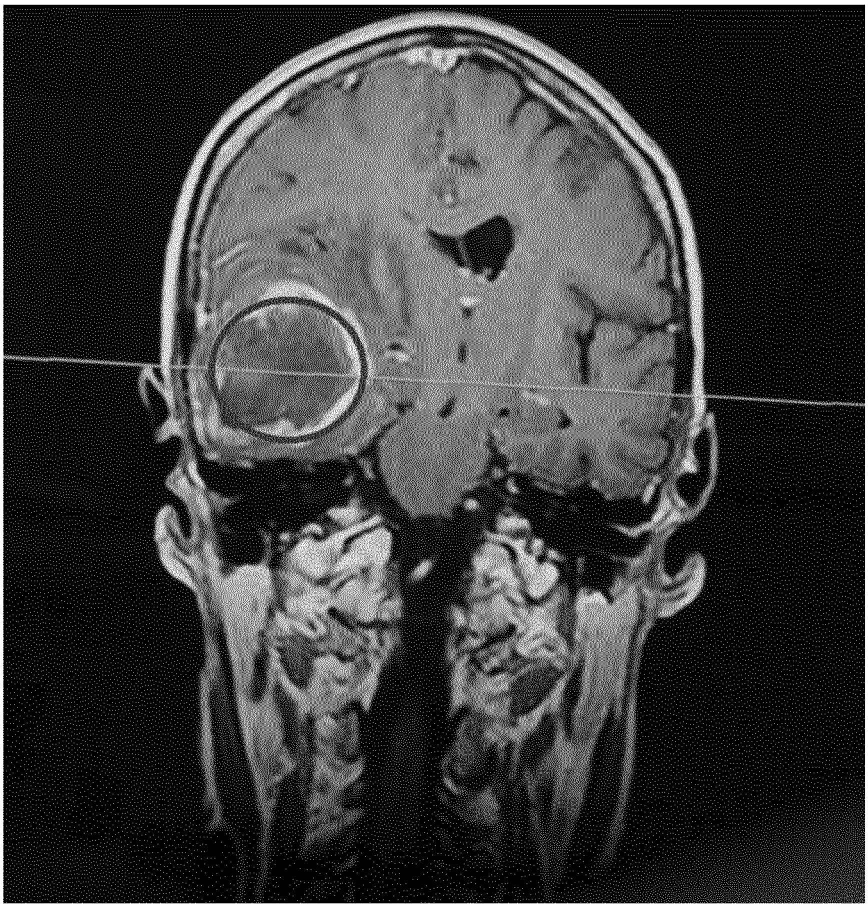

FIG. 7B

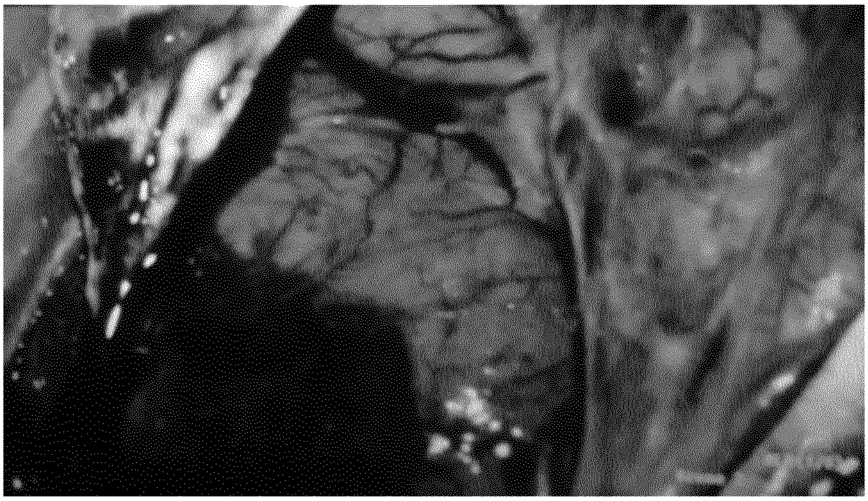

## INTERNATIONAL SEARCH REPORT

International application No. **PCT/KR2021/015034**

### A. CLASSIFICATION OF SUBJECT MATTER

**A61K 49/00**(2006.01)i; **A61K 8/41**(2006.01)i; **A61K 8/9789**(2017.01)i; **A61K 8/44**(2006.01)i; **A61K 8/49**(2006.01)i; **A61K 8/9728**(2017.01)i; **A61K 8/98**(2006.01)i

According to International Patent Classification (IPC) or to both national classification and IPC

### B. FIELDS SEARCHED

Minimum documentation searched (classification system followed by classification symbols)

A61K 49/00(2006.01); A61K 31/197(2006.01); A61K 41/00(2006.01); A61K 8/97(2006.01); A61K 8/9728(2017.01); A61K 8/9789(2017.01); A61N 5/06(2006.01); A61Q 19/00(2006.01); C07K 14/705(2006.01); C07K 16/30(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 5-아미노레불린산 수화염화물(5-aminolevulinic acid hydrochloride), 발효 더덕 추출물(fermented deodeok extract), 동충하초 추출물(cordyceps extract), 폴리데옥시리보뉴클레오티드 나트륨(polydeoxyribonucleotide sodium), 히아루로니다제(hyaluronidase), L-카르니틴(L-carnitine), 카페인(caffeine), 오레가노 잎 오일(origanum vulgare leaf oil), 아스코르브산(vitamin C), 알란토인(allantion), 여드름(acne), 인돌-3-아세트산(indole-3-acetic acid), 폴리에틸렌이민(polyethylenimine), 자기조립리간드입자(self-assembled ligand particle), 광역학 치료(photodynamic therapy)

### C. DOCUMENTS CONSIDERED TO BE RELEVANT

| Category* | Citation of document, with indication, where appropriate, of the relevant passages | Relevant to claim No. |
|---|---|---|
| A | US 2013-0066256 A1 (PHOTOCURE ASA) 14 March 2013 (2013-03-14) See paragraphs [0067]-[0071] and [0083]; and claims 1-3. | 1-5 |
| A | KR 10-2165525 B1 (PARK, Seong Eun) 14 October 2020 (2020-10-14) See entire document. | 1-5 |
| A | KR 10-2009-0002678 A (LG HOUSEHOLD & HEALTH CARE LTD.) 09 January 2009 (2009-01-09) See entire document. | 1-5 |
| A | KR 10-2010-0095443 A (PHOTOCURE ASA) 30 August 2010 (2010-08-30) See paragraphs [0065], [0069] and [0071]; and claims 1 and 28. | 1-5 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **25 January 2022** | **25 January 2022** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**

International application No.
**PCT/KR2021/015034**

**C. DOCUMENTS CONSIDERED TO BE RELEVANT**

| Category* | Citation of document, with indication, where appropriate, of the relevant passages | Relevant to claim No. |
|---|---|---|
| A | US 2018-0112006 A1 (THE GENERAL HOSPITAL CORPORATION) 26 April 2018 (2018-04-26)<br>See entire document. | 1-5 |
| A | KR 10-2020-0081621 A (SEOUL NATIONAL UNIVERSITY R&DB FOUNDATION) 08 July 2020 (2020-07-08)<br>See entire document. | 1-5 |
| PX | KR 10-2265592 B1 (KIM, Jin-Wang et al.) 17 June 2021 (2021-06-17)<br>See entire document.<br>(*This document is the published patent of a priority application of the present international application.) | 1-2 |

Form PCT/ISA/210 (second sheet) (July 2019)

**INTERNATIONAL SEARCH REPORT**
**Information on patent family members**

International application No.
**PCT/KR2021/015034**

| Patent document cited in search report | Publication date (day/month/year) | Patent family member(s) | Publication date (day/month/year) |
|---|---|---|---|
| US 2013-0066256 A1 | 14 March 2013 | CN 101056626 A | 17 October 2007 |
| | | CN 101056626 B | 14 December 2011 |
| | | EP 1814534 A1 | 08 August 2007 |
| | | EP 1814534 B1 | 02 June 2010 |
| | | JP 2008-519812 A | 12 June 2008 |
| | | JP 5134370 B2 | 30 January 2013 |
| | | US 2008-0188558 A1 | 07 August 2008 |
| | | US 8546447 B2 | 01 October 2013 |
| | | WO 2006-051269 A1 | 18 May 2006 |
| KR 10-2165525 B1 | 14 October 2020 | None | |
| KR 10-2009-0002678 A | 09 January 2009 | KR 10-1456044 B1 | 04 November 2014 |
| | | KR 10-1456061 B1 | 03 November 2014 |
| | | KR 10-1456062 B1 | 04 November 2014 |
| | | KR 10-2013-0135223 A | 10 December 2013 |
| | | KR 10-2014-0002580 A | 08 January 2014 |
| KR 10-2010-0095443 A | 30 August 2010 | CN 101896203 A | 24 November 2010 |
| | | CN 104306968 A | 28 January 2015 |
| | | EP 2231188 A2 | 29 September 2010 |
| | | EP 2684573 A2 | 15 January 2014 |
| | | EP 2684573 A3 | 24 September 2014 |
| | | JP 2011-507808 A | 10 March 2011 |
| | | JP 5686603 B2 | 18 March 2015 |
| | | US 2011-0020441 A1 | 27 January 2011 |
| | | US 2015-0031761 A1 | 29 January 2015 |
| | | WO 2009-074811 A2 | 18 June 2009 |
| | | WO 2009-074811 A3 | 06 August 2009 |
| US 2018-0112006 A1 | 26 April 2018 | EP 3283529 A1 | 21 February 2018 |
| | | EP 3283529 A4 | 07 November 2018 |
| | | WO 2016-168601 A1 | 20 October 2016 |
| KR 10-2020-0081621 A | 08 July 2020 | KR 10-2179530 B1 | 18 November 2020 |
| KR 10-2265592 B1 | 17 June 2021 | None | |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 101322259
- KR 101638664
- KR 101135147
- KR 101622031
- KR 101659855
- KR 101183732